Europäisches Patentamt

European Patent Office

Office européen des brevets

(11) Publication number: **0 424 130 B1**

(19)

# EUROPEAN PATENT SPECIFICATION

(45) Date of publication of patent specification: **01.02.95**  (51) Int. Cl.⁶: **C12N  11/00**, C12P 7/64

(21) Application number: **90311416.3**

(22) Date of filing: **18.10.90**

(54) **Supported enzyme.**

(30) Priority: **20.10.89 EP 89202659**
**06.09.90 GB 9019437**

(43) Date of publication of application:
**24.04.91 Bulletin  91/17**

(45) Publication of the grant of the patent:
**01.02.95 Bulletin  95/05**

(84) Designated Contracting States:
**AT BE CH DE DK ES FR GB GR IT LI NL SE**

(56) References cited:
**EP-A- 0 122 209**
**EP-A- 0 140 542**
**EP-A- 0 320 132**
**EP-A- 0 322 213**
**WO-A-89/06278**

**PATENT ABSTRACTS OF JAPAN, vol. 12, no.**
**15 (C-469)(2862), 16 January 1988**

**PATENT ABSTRACTS OF JAPAN, vol. 7, no.**
**167 (C-177)(1312), 22 July 1983**

**PATENT ABSTRACTS OF JAPAN, vol. 12, no.**
**373 (C-534)(3220), 6 October 1988**

**PATENT ABSTRACTS OF JAPAN, vol. 7, no.**

**147 (C-173)(1292), 28 June 1983**

**PATENT ABSTRACTS OF JAPAN, vol. 13, no.**
**451 (C-643)(3799), 11 October 1989**

(73) Proprietor: **UNICHEMA CHEMIE BV**
**Buurtje 1**
**NL-2802 BE Gouda (NL)**

(72) Inventor: **Bosley, John Anthony**
**43 Kettering Road,**
**Islip**
**Kettering,**
**Northamptonshire NN14 3JT (GB)**
Inventor: **PEILOW, Alan David**
**17, Lancaster Close**
**Wollaston, Northamptonshire NN9 7PD (GB)**

(74) Representative: **Bot, David Simon Maria et al**
**Unilever N.V.**
**Patent Division**
**P.O. Box 137**
**NL-3130 AC Vlaardingen (NL)**

**EP 0 424 130 B1**

**Description**

The invention relates to a supported enzyme, the preparation and use thereof. More in particular the invention relates to lipase suported on a carrier material.

Lipases supported on a carrier material are valuable materials for carrying out chemical reactions, enabling the enzyme to be used many times. They are known in the art and are normally prepared by adsorption onto a suitable carrier material from an aqueous solution of lipase, followed by drying.

One of the disadvantages such materials have is that with the usual adsorption technique onto a carrier, an appreciable percentage of the activity of the lipase is lost.

Several hypotheses for this phenomenon have been offered, such as, eg., that by adsorption onto the carrier the conformation of the enzyme is altered, which then leads to a partial loss of its activity.

More specifically, enzymes, in particularly lipase, on support materials are known from EP-A-322213 (Unilever) which discloses inter alia the preparation of fatty acid esters using a lipase directly physically attached by adsorption onto a hydrophobic, porous, solid, support material.

US 4798793 and US 4818695 (Novo) describe the immobilisation of lipases on weak anion exchange resins.

The present invention provides a lipase supported on a carrier material, characterised in that the carrier material has a substantial coating of a non-lipase protein and an at least partial coating of lipase, both the non-lipase protein and the lipase being non-covalently bound to the carrier material.

Supported lipase in accordance with the invention has an appreciably higher activity than the equivalent loading of lipase on a carrier material not also bearing a non-lipase protein, and consequently more of the original activity of the lipase is retained.

Particulate carrier materials are preferred, especially those having a particle size of from 100 to 2000 micron. The carrier material may also be in the form of a membrane.

Preferably the carrier material is porous, having an average pore diameter greater than 50 nanometers. Preferably, the carrier material is selected from hydrophobic materials and ion-exchange resins.

When the carrier material is a hydrophobic material this may be selected from polypropylene, polyolefin, polystyrene, polyacrylate(ester), inorganic materials like silicate, silica, glass etc. or combinations thereof. For this embodiment, materials like silica etc. which are normally hydrophilic have to be treated with a suitable compound eg. a silane to render them hydrophobic.

When the carrier material is an ion-exchange resin, this may be selected from ion-exchange resins based on polystyrene, polyacrylate, phenol-formaldehyde resins and silicas. Ideally, the ion-exchange resin is an anion exchanger, especially a macroporous weak anion exchange resin. Suitable examples include phenol-formaldehyde, polystyrenic, and styrene-DVB resins such as are available under the Trade Marks DUOLITE ES568 and AMBERLYST A21.

The non-lipase protein used to coat the carrier particles is preferably a water-soluble protein such as ovalbumin, gelatin, bovine serum albumin and/or sodium caseinate. This coating with protein material is so applied that the surface area of the carrier is substantially coated with up to a monolayer of the non-lipase protein. In the coating process pore volume of the support material and the amount of and concentration of non-lipase protein aqueous solution are so chosen that a substantially complete coating of the surface of the support material is aimed at. A substantially complete coating is understood as a coating of at least 50%, preferably over 85% of the available surface area. Simultaneously or subsequently the carrier material is at least partially coated with lipase.

The lipase which is used in the practice of this invention can be obtained by culturing a suitable microorganism and subsequent isolation of the enzyme.

Suitable microorganisms for this purpose belong to the genera Mucor, Aspergillus, Rhizopus, Pseudomonas, Candida, Humicola, Thermomyces and Penicillium.

It is further preferred that the lipase supported on the carrier provided by the present invention is present at 0.5 to 75% by weight of the non-lipase protein. More preferably the lipase is present at 1 to 50% by weight of the non-lipase protein. After adsorption of the lipase the available surface area of the carrier is coated with up to two layers (up to a bilayer) of proteinaceous materials.

In a further aspect the invention provides a process for preparing a lipase supported on a carrier material, characterised by non-covalently substantially coating the carrier material with a non-lipase protein and, simultaneously or subsequently, non-covalently at least partially coating the carrier material with lipase.

The lipase on carrier material provided by the present invention can conveniently be prepared by treating a hydrophobic carrier material with a solution of a non-lipase protein as to cover the available surface area of the carrier particles substantially with up to a monolayer of non-lipase protein and depositing on this coated carrier at least a partial coating of lipase.

2

The lipase on carrier material provided by the present invention can be used with advantage in processes for preparing an ester by interesterification by heating and reacting carboxylic acid and an ester in the presence of a lipase supported on a carrier as provided by the present invention. Preparation techniques involving the use of lipase on carrier material permit the repeated use of the lipase material eg. in continuous methods such as in a fixed bed or pipe reactor or in a batchwise method such as a stirred tank reactor.

The lipase on carrier material according to the present invention can also be used in the preparation of esters by esterification by reacting an alcohol and a carboxylic acid in the presence of a lipase supported on a carrier as provided by the present invention. The invention is illustrated by the following examples. The assays used therein were carried out as described below.

**Assays**

A. Esterification

The catalyst (5-20 mg, depending on loading) was placed in a vial and a water-saturated mixture containing 5.88 g oleic acid (92%, ex BDH) and 2.70 g octan-l-ol (GPR grade, ex BDH) were added. The vial was sealed and placed on a shaker in a water bath at 50°C for 30 minutes, shaking at 200 strokes/minute. An amount (0.1 millilitre) was removed and immediately eluted down a small alumina column (basic, activity 2) with diethyl ether, together with a solution of methyl stearate (2.5 mg) as an internal standard. The diethyl ether was then removed by evaporation and replaced by petroleum ether (4.0 ml, bp 60-80°C). The ratio of octyl oleate to methyl stearate was then determined by GLC. From this ratio the rate of ester formation was calculated and the efficiency expressed by dividing this rate by the theoretical lipase loading.

B. Interesterification

The catalyst (0.5-1.0 g) was packed in a glass column 15 mm in diameter, together with 4.0 g wet ID silica gel (ex Joseph Crosfields & Sons, Warrington, U.K.) containing 3.2 g water as a pre-column. Water-saturated feedstock comprising 1 part high-oleate sunflower oil, 0.7 parts lauric acid (98%, ex Unichema), and 4 part petroleum ether (bp 100-120°C) by weight, was pumped through the column at a flow rate of 25 ml/hour. The column temperature was maintained at 50°C using a water jacket. The amount of lauric acid incorporated into the triglyceride was determined by FAME/GC analysis. The column was run continuously for 5 days, the activity obtained on day 2 was used in the examples.

The activity is calculated using the following equation;

$$\text{Activity} = \frac{-\ln(1-DC) \times \text{flowrate}}{\text{catalyst wt. (g)}} \quad (\text{g.triglyceride/hour/g.catalyst})$$

$$\text{flowrate} = \text{g. triglyceride/hour}$$

$$DC = \frac{\% \,(\text{lauric incorporated} - \text{initial content})}{\% \,(\text{equilibrium content} - \text{initial content})}$$

$$DC = \text{Degree of conversion}$$

For these examples, initial lauric content = 0

equilibrium content = 31.3%

Efficiency is calculated by dividing the activity obtained by the theoretical lipase loading. This is expressed in units of mgTg/hour/thousand lipase units (mgTg/hour/KLU).

Example 1

To 2.0 g macroporous polypropylene particles (mean pore diameter 139 nanometers, particle size 0.2-0.4 millimetre) were added 6.0 ml of ethanol with shaking to ensure that all the polymer particles were wetted. To this slurry was added 54 ml 0.01M sodium phosphate buffer (pH7) with stirring to ensure complete mixing. A further quantity of 200 ml of the same phosphate buffer containing 516 mg of the pretreatment protein (see table below) was added and the mixture gently stirred for 24 hours at 20°C. The treated support was then separated from the solution by filtration and washed four times with the same phosphate buffer (100 ml each). 1.0g of this material was suspended in 40 ml of the same phosphate buffer.

To the suspended pre-treatment support were added 100 ml of the same phosphate buffer containing a quantity of Mucor miehei lipase (10,000 LU/ml) as further described in the table below. The mixture was gently stirred for 24 hours at 20°C. The enzyme loading achieved was calculated from the loss of enzyme activity from the solution as determined by the rate of hydrolysis of glyceryl tributyrate. The resultant lipase on carrier was separated by filtration, washed twice with the same phosphate buffer (200 ml), washed once with distilled water (100 ml) and dried under vacuum at 20°C.

Table 1

| Pretreatment Protein[1] | Lipase Solution ml | Theoretical Loading LU/g | Efficiency | |
|---|---|---|---|---|
| | | | A | B |
| None | 1.7 | 18,600 | 1.5 | 48.4 |
| Sodium caseinate | 2.0 | 18,100 | 3.8 | 204.4 |
| Ovalbumin | 2.0 | 17,800 | 4.4 | 236.0 |
| Bovine serum albumin | 5.0 | 26,500 | 5.2 | 245.3 |
| None | 4.9 | 49,700 | 2.8 | 110.7 |
| Ovalbumin | 5.0 | 45,300 | 4.4 | 287.0 |
| Sodium caseinate | 5.0 | 46,500 | 5.3 | - |
| None | 8.5 | 78,200 | 3.3 | - |
| Sodium caseinate | 8.0 | 78,400 | 5.4 | - |
| A = Esterification, micromoles/hour/LU | | | | |
| B = Interesterification, mgTg/hour/KLU | | | | |

[1] Sodium caseinate (spray bland, 94% protein, ex De Melkindustrie Veghel (DMV), Netherlands)
Ovalbumin (Grade V, ex Sigma Chemical Ltd, Poole, England)
Bovine serum albumin (98-99%, ex Sigma Chemical Ltd, Poole, England)

Example 2

The procedure of Example 1 was repeated except that the lipase solution used was from Humicola sp. - (ex Novo Industries Denmark, 50,000 lipase units per ml). The results are tabulated below.

Table 2

| Pretreatment Protein | Lipase Solution ml | Theoretical Loading LU/g | Efficiency | |
|---|---|---|---|---|
| | | | A | B |
| None | 1.0 | 62,300 | 0.3 | 93.1 |
| Ovalbumin | 1.3 | 54,600 | 0.8 | 227.1 |
| None | 3.1 | 174,000 | 0.7 | - |
| Ovalbumin | 6.1 | 173,400 | 1.0 | - |

Example 3

The procedure of Example 3 was repeated except that the lipase used was from Rhizopus niveus (ex Amano N, Amano Japan, 4,500 lipase units per gram). The lipase was dissolved in 100 ml phosphate buffer before addition to the treated support.

| Pretreatment Protein | Lipase Added g | Theoretical Loading LU/g | Efficiency |
|---|---|---|---|
| | | | A |
| None | 3.125 | 11,500 | 2.6 |
| Ovalbumin | 3.300 | 12,000 | 3.5 |

Example 4

5.0 g of controlled pore glass beads (ex Sigma Chemical Ltd, Poole, England, mean pore diameter 187 nanometers, surface area 11 m$^2$/g) were dried in an oven at 105°C for 15 minutes. After cooling over phosphorous pentoxide, a solution of dichloromethylsilane (16 ml) in 1,1,1,-trichloroethane (64 ml) was added and the beads stirred. After 1½ hours the beads were filtered, washed with 1,1,1,-trichlorothane and dried under vacuum at 20°C.

The procedure of Example 1 was repeated except that 2.0 g hydrophobic glass beads were wetted with 100 ml ethanol before addition of the pretreatment protein solution. 1 g of the pre-treated beads were suspended in 50 ml buffer:ethanol (9:1) mixture prior to addition of the lipase.

| Pretreatment Protein | Lipase Added g | Theoretical Loading LU/g | Efficiency |
|---|---|---|---|
| | | | A |
| None | 2.5 | 24,200 | 3.0 |
| Ovalbumin | 1.8 | 29,400 | 5.4 |

Example 5

The procedure of Example 1 was repeated except that the support used was a hydrophobic macroporous polystyrene particle (ex National Starch & Chemical Corp, Bridgewater, USA, mean pore diameter 1,660 nanometers, surface area 11 m$^2$/g). The initial wetting was achieved by adding 10 ml ethanol to 2.0 g support followed by 50 ml phosphate buffer.

| Pretreatment Protein | Lipase Added g | Theoretical Loading LU/g | Efficiency |
|---|---|---|---|
| | | | A |
| None | 0.39 | 3,700 | 0.7 |
| Ovalbumin | 0.35 | 3,500 | 2.2 |

Example 6

To 2.0 g moist Duolite ES568 weak anion exchange resin (29% moisture) was added 6.0 ml ethanol with shaking to ensure all the resin particles were wetted. To this slurry was added 54 ml 0.01M sodium phosphate buffer (pH7) with stirring to ensure complete mixing. A further quantity of 140 ml of the same phosphate buffer containing 516 mg of the pretreatment protein was added and the mixture gently stirred for 16 hours at 20°C. The resin was allowed to settle and the supernatant solution was decanted off. The treated resin was then washed with 3 x 100 ml phosphate buffer and separated by filtration. To the washed treated resin was added 70 ml phosphate buffer containing a quantity of Mucor miehei lipase (10,000 LU/ml) as given in the table below. The mixture was gently stirred for 16 hours at 20°C. The enzyme loading achieved was calculated from the loss of enzyme activity from the solution as determined by hydrolysis of tributyrin. The resultant immobilised lipase was collected by filtration, washed with 2 x 200 ml phosphate buffer followed by 100 ml distilled water, and dried under vacuum at 20°C.

| Pretreatment Protein[1] | Lipase Solution ml. | Theoretical Loading LU/g | Efficiency[2] |
|---|---|---|---|
| | | | A |
| None | 0.6 | 4,170 | 2.0 |
| Bovine serum albumin | 6.2 | 5,770 | 3.6 |
| Ovalbumin | 6.2 | 4,370 | 4.2 |
| Sodium caseinate | 6.2 | 3,660 | 4.4 |

[1] Bovine serum albumin, 98-99%, Sigma Chemical Co., Poole, GB
Ovalbumin, Grade V, Sigma Chemical Co., Poole, GB
Sodium caseinate, Spray bland, 94% protein, DMV, Netherlands
[2] Esterification, micromoles/hour/LU.

Example 7

The procedure of Example 6 was repeated except the lipase used was from Humicola sp. (NOVO-Nordisk, 50,000 LU/ml.).

| Pretreatment Protein | Lipase Solution ml. | Theoretical Loading LU/g | Efficiency[1] |
|---|---|---|---|
| | | | A |
| None | 1.2 | 39,900 | 0.23 |
| Ovalbumin | 1.2 | 33,700 | 0.72 |

[1] Esterification, micromoles/hour/LU.

Example 8

A 15.0 g sample of moist weak anion exchange resin, Duolite ES568, (29% moisture) was placed in an extraction thimble and washed by soxhlet extraction with propan-2-ol for 16 hours. This was then washed with 2 x 200 ml ethanol and dried under vacuum at 20°C. This procedure was also repeated with a 15.0 g sample of another weak anion exchange resin, Amberlyst A-21.

6

The procedure of Example 6 was then followed except that the starting material was dried washed resin as prepared above. The pretreatment protein used was ovalbumin and the volume of Mucor miehei lipase solution used as 0.8 ml.

| Support | Pretreatment | Theoretical Loading LU/g | Efficiency[1] |
|---------|--------------|--------------------------|---------------|
| Duolite ES568 | No | 4,140 | 1.9 |
| Duolite ES568 | Yes | 3,740 | 5.9 |
| Amberlyst A-21 | No | 3,430 | 0.7 |
| Amberlyst A-21 | Yes | 2,810 | 5.5 |

[1] Esterification, micromoles/hour/LU

Example 9

The procedure of Example 6 was repeated except that the supports used were a strong anion exchange silica, Spherosil QMA, and a weak anion exchange silica, Spherosil DEA. No ethanol was used to wet these ion exchange silicas prior to the addition of the ovalbumin pretreatment protein. The volume of Mucor miehei lipase solution added was 0.8 ml in 200 ml phosphate buffer.

| Support | Pretreatment | Theoretical Loading LU/g | Efficiency[1] |
|---------|--------------|--------------------------|---------------|
| Spherosil QMA | No | 4,180 | 1.3 |
| Spherosil QMA | Yes | 4,190 | 5.2 |
| Spherosil DEA | No | 3,670 | 1.6 |
| Spherosil DEA | Yes | 2,140 | 4.1 |

[1] Esterification, micromoles/hour/LU

Example 10

The procedure of Example 8 was repeated using Duolite ES568 as support except that the pretreatment protein was sodium caseinate. The volume of Mucor miehei lipase solution added was 1.0 ml.

| Pretreatment | Theoretical Loading LU/g | Efficiency[1] |
|--------------|--------------------------|---------------|
| No | 2,410 | 46 |
| Yes | 1,680 | 89 |

[1] Interesterification, mgTg/hour/KLU

Example 11

The proceedure of Example 8 was repeated using Amberlyst A-21 as support except that the lipase used was from Humicola sp., the volume used was 0.8 ml.

| Pretreatment | Theoretical Loading LU/g | Efficiency[1] |
|--------------|--------------------------|---------------|
| No | 11,900 | 81 |
| Yes | 11,600 | 169 |

[1] Interesterification, mgTg/hour/KLU

Example 12

To the hydrophobic macroporous polypropylene particles (2.0 g., Accurel EP100, ENKA) was added 20 mls absolute ethanol with stirring to ensure all the polymer particles were wetted. To this was added 200 mls 0.01M sodium phosphate buffer solution (pH 7) with stirring to ensure complete mixing. Excess solution, approximately 190 mls, was decanted off and a further 100 ml aliquot of buffer containing a mixture of lipase and non-lipase protein added. The mixture was gently stirred at room temperature. The adsorption of lipase to the porous support was monitored by loss of activity from the solution. The theoretical enzyme loadings achieved and the efficiencies obtained are given in the table below.

| a) Lipase = Mucor miehei, 10,000 LU/ml. ex-Novo Industries. | | | | |
| Lipase Solution mls. | Ovalbumin mg. | Theoretical Loading LU/g | Efficiency[1] | |
| | | | A | B |
| 3.3 | 0 | 18,600 | 1.9 | 46 |
| 4.0 | 32 | 22,800 | 2.3 | 136 |
| 4.0 | 66 | 22,800 | 2.3 | 145 |
| 4.0 | 130 | 22,500 | 2.5 | 178 |
| 4.0 | 258 | 16,400 | 3.1 | 220 |
| 4.0 | 516 | 16,400 | 3.1 | 195 |
| 4.0 | 2580 | 16,400 | 3.7 | 211 |
| | Sodium Caseinate mg. | | | |
| 4.0 | 516 | 18,000 | 6.6 | 233 |
| | BSA, mg. | | | |
| 4.0 | 516 | 19,500 | 3.9 | 226 |
| | Ovalbumin mg. | | | |
| 17.0 | 0 | 77,400 | 3.0 | 105 |
| 19.0 | 516 | 77,600 | 5.2 | 294 |
| | Sodium Caseinate mg. | | | |
| 19.0 | 516 | 76,600 | 6.4 | 305 |

[1] A = Esterification, micromoles/hour/LU.
B = Interesterification, mgTg/hour/KLU.

| b) Lipase = Humicola sp., 50,000 LU/ml., ex-Novo Industries. | | | | |
| Lipase solution mls. | Ovalbumin mg. | Theoretical Loading LU/g | Efficiency[1] | |
| | | | A | B |
| 1.0 | 0 | 22,600 | 0.2 | 122 |
| 1.3 | 516 | 30,600 | 1.1 | 225 |

[1] A = Esterification, micromoles/hour/LU.
B = Interesterification, mgTg/hour/KLU.

Example 13

To 1.0g macroporous polypropylene particles (mean pore diameter 139 nanometres, particle size 0.2-0.4 millimetre) was added 3.0 ml ethanol with shaking to ensure all the polymer particles were wetted. To this slurry was added 27 ml 0.01M sodium phosphate buffer (pH 7) and the mixture warmed to 50°C in a water bath. To this was added a solution of 0.258 g gelatin (250 bloom, ex Fluka AG, Switzerland) in 100 ml 0.01M sodium phosphate buffer (pH 7) at 50°C with stirring. The mixture was left stirring in a water bath at 50°C for 16 hours. The particles were then washed with 4 aliquots of 50 ml of the same buffer at 50°C and then most of the free liquid removed by filtration. The volume of the suspension was then made up to 40 ml with the same phosphate buffer and a solution of Mucor miehei lipase (2 ml @ 10,000 LU/ml) in 100 ml of the buffer added. The mixture was gently stirred at 20°C for 22 hours. The adsorption of lipase to the support was monitored by loss of activity from the solution and was found to be 19,400 LU/g. The resultant immobilised lipase was separated by filtration and washed twice with 200 ml of the buffer followed by once with 100 ml distilled water. The product was dried under vacuum at 20°C.

| Pretreatment | Theoretical Loading LU/g | Efficiency[1] |
|---|---|---|
| None | 18,600 | 48.4 |
| Gelatin | 19,400 | 165.2 |

[1] Interesterification, mgTg/hour/KLU

## Claims

1.  A lipase supported on a carrier material, characterised in that the carrier material has a substantial coating of a non-lipase protein and an at least partial coating of lipase, both the non-lipase protein and the lipase being non-covalently bound to the carrier material.

2.  A lipase supported on a carrier according to claim 1, wherein the carrier material is hydrophobic.

3.  A lipase supported on carrier according to claim 2, in which the hydrophobic carrier material is polyolefin, polystyrene, polyacrylate, silicate, silica or glass.

4.  A lipase supported on a carrier according to claim 1, 2 or 3, wherein the carrier material is an ion-exchange resin.

5.  A lipase supported on a carrier according to claim 4, wherein the ion-exchange resin is selected from ion-exchange resins based on polystyrene, polyacrylate, phenol-formaldehyde resins and silicas.

6.  A lipase supported on a carrier according to claim 5, wherein the ion-exchange resin is an anionic ion-exchange resin.

7.  A lipase supported on carrier according to any one of the preceding claims, wherein the carrier material has an average pore diameter greater than 50 nanometers.

8.  A lipase supported on carrier according to any one of the preceding claims, wherein the non-lipase protein comprises ovalbumin, gelatin, bovine serum albumin and/or sodium caseinate.

9.  A lipase supported on carrier according to any one of the preceding claims wherein the lipase is present at 0.5 to 75%, preferably 1 to 50% by weight of the non-lipase protein.

10. A process for preparing a lipase supported on a carrier material, characterised by non-covalently substantially coating the carrier material with a non-lipase protein and, simultaneously or subsequently, non-covalently at least partially coating the carrier material with lipase.

11. A process according to claim 10, wherein the carrier material is treated with a solution of the non-lipase protein so as to cover the carrier material substantially with the non-lipase protein.

12. A process for preparing an ester by interesterification by heating and reacting a carboxylic acid and an ester in the presence of a lipase supported on a carrier as defined in any one of claims 1 to 9.

13. A process for preparing an ester by esterification by heating and reacting a carboxylic acid and an alcohol in the presence of a lipase supported on a carrier as defined in any one of claims 1 to 9.

**Patentansprüche**

1. Lipase auf einem Trägermaterial, dadurch gekennzeichnet, daß das Trägermaterial eine substantielle Beschichtung eines Nicht-Lipaseproteins und mindestens eine partielle Beschichtung von Lipase aufweist, wobei sowohl das Nicht-Lipaseprotein als auch die Lipase nicht-kovalent an das Trägermaterial gebunden sind.

2. Lipase auf einem Trägermaterial nach Anspruch 1, worin das Trägermaterial hydrophob ist.

3. Lipase auf einem Trägermaterial nach Anspruch 2, in welcher das hydrophobe Trägermaterial Polyolefin, Polystyrol, Polyacrylat, Silicat, Siliciumoxid oder Glas ist.

4. Lipase auf einem Trägermaterial nach Anspruch 1, 2 oder 3, worin das Trägermaterial ein Ionenaustauscherharz ist.

5. Lipase auf einem Trägermaterial nach Anspruch 4, worin das Ionenaustauscherharz ausgewählt ist aus Ionenaustauscherharzen auf der Basis von Polystyrol, Polyacrylat, Phenol-Formaldehyd-Harzen und Siliciumoxiden.

6. Lipase auf einem Trägermaterial nach Anspruch 5, worin das Ionenaustauscherharz ein anionisches Ionenaustauscherharz ist.

7. Lipase auf einem Trägermaterial nach irgendeinem der vorhergehenden Ansprüche, worin das Trägermaterial einen durchschnittlichen Porendurchmesser von mehr als 50 nm hat.

8. Lipase auf einem Trägermaterial nach irgendeinem vorhergehenden Anspruch, worin das Nicht-Lipaseprotein Ovalbumin, Gelatine, Rinderserumalbumin und/oder Natriumkaseinat umfaßt.

9. Lipase auf einem Trägermaterial nach irgendeinem vorhergehenden Anspruch, worin die Lipase in einer Menge von 0,5 bis 75 Gew.-%, vorzugsweise 1 bis 50 Gew.-%, des Nicht-Lipaseproteins vorliegt.

10. Verfahren zur Herstellung einer Lipase auf einem Trägermaterial, gekennzeichnet durch das nicht-kovalente, substantielle Beschichten des Trägermaterials mit einem Nicht-Lipaseprotein und das gleichzeitige oder anschließende, nicht-kovalente, partielle Beschichten des Trägermaterials mit Lipase.

11. Verfahren nach Anspruch 10, bei dem das Trägermaterial mit einer Lösung des Nicht-Lipaseproteins behandelt wird, um so das Trägermaterial im wesentlichen mit dem Nicht-Lipaseprotein zu bedecken.

12. Verfahren zur Herstellung eines Esters mittels gegenseitiger Veresterung durch Erhitzen und Umsetzen einer Carbonsäure und eines Esters in Gegenwart einer Lipase auf einem Trägermaterial, wie sie in irgendeinem der Ansprüche 1 bis 9 definiert ist.

13. Verfahren zur Herstellung eines Esters mittels Veresterung durch Erhitzen und Umsetzen einer Carbonsäure und eines Alkohols in Gegenwart einer Lipase auf einem Trägermaterial, wie sie in irgendeinem der Ansprüche 1 bis 9 definiert ist.

**Revendications**

1. Lipase sur un support, caractérisée en ce que la matière de support porte un enrobage substantiel d'une protéine autre qu'une lipase et au moins un enrobage partiel de lipase, aussi bien la protéine autre que la lipase que la lipase étant liées de façon non covalente à la matière de support.

2. Lipase sur un support selon la revendication 1, dans laquelle le support est hydrophobe.

3. Lipase sur un support selon la revendication 2, dans laquelle le support hydrophobe est une polyoléfine, un polystyrène, un polyacrylate, un silicate, une silice ou le verre.

**4.** Lipase sur un support selon la revendication 1, 2 ou 3, dans laquelle la matière de support est une résine échangeuse d'ions.

**5.** Lipase sur un support selon la revendication 4, dans laquelle la résine d'échange d'ions est choisie parmi les résines échangeuses d'ions à base de polystyrène, polyacrylate, résines phénol-formaldéhyde et silices.

**6.** Lipase sur un support selon la revendication 5, dans laquelle la résine échangeuse d'ions est une résine échangeuse d'anions.

**7.** Lipase sur un support selon l'une quelconque des revendications précédentes, dans laquelle la matière de support présente un diamètre moyen de pores de plus de 50 nm.

**8.** Lipase sur un support selon l'une quelconque des revendications précédentes, dans laquelle la protéine autre que la lipase comprend l'ovalbumine, une gélatine, l'albumine de sérum bovin et/ou le caséinate de sodium.

**9.** Lipase sur un support selon l'une quelconque des revendications précédentes, dans laquelle la lipase est présente à raison de 0,5 à 75%, de préférence de 1 à 50% en poids de la protéine autre que la lipase.

**10.** Procédé de préparation d'une lipase sur un support, caractérisé en ce qu'on en robe substantiellement, selon un mode non covalent la matière de support avec une protéine autre qu'une lipase et, simultanément ou ultérieurement, on enrobe la matière de support au moins partiellement selon un mode non covalent avec une lipase.

**11.** Procédé selon la revendication 10, dans lequel on traite la matière de support avec une solution de protéine autre que la lipase de façon à recouvrir substantiellement la matière de support avec la protéine autre que la lipase.

**12.** Procédé de préparation d'un ester par interestérification consistant à chauffer et à faire réagir un acide carboxylique et un ester en présence d'une lipase sur un support selon l'une quelconque des revendications 1 à 9.

**13.** Procédé de préparation d'un ester par estérification consistant à chauffer et à faire réagir un acide carboxylique et un alcool en présence d'une lipase sur un support selon l'une quelconque des revendications 1 à 9.